(19)

**Europäisches Patentamt**
**European Patent Office**
**Office européen des brevets**

(11) **EP 4 513 396 A1**

(12) **EUROPEAN PATENT APPLICATION**

(43) Date of publication:
**26.02.2025 Bulletin 2025/09**

(21) Application number: **24194652.4**

(22) Date of filing: **14.08.2024**

(51) International Patent Classification (IPC):
*G06Q 10/04* (2023.01)   *G06Q 10/06* (2023.01)
*G06Q 50/04* (2012.01)   *G06Q 50/06* (2024.01)

(52) Cooperative Patent Classification (CPC):
**G06Q 10/04; G06Q 10/06; G06Q 50/04; G06Q 50/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.08.2023 JP 2023137610**

(71) Applicant: **Yokogawa Electric Corporation Tokyo 180-8750 (JP)**

(72) Inventors:
- **KOBAYASHI, Yasunori**
  **Musashino-shi, Tokyo, 180-8750 (JP)**
- **SHINOHARA, Junichi**
  **Musashino-shi, Tokyo, 180-8750 (JP)**
- **ITOU, Satoshi**
  **Musashino-shi, Tokyo, 180-8750 (JP)**
- **ENDO, Makoto**
  **Musashino-shi, Tokyo, 180-8750 (JP)**

(74) Representative: **Stöckeler, Ferdinand et al Schoppe, Zimmermann, Stöckeler Zinkler, Schenk & Partner mbB Patentanwälte Radlkoferstrasse 2 81373 München (DE)**

(54) **INFORMATION PROCESSING METHOD, INFORMATION PROCESSING APPARATUS, INFORMATION PROCESSING SYSTEM, AND PROGRAM**

(57)     Plant operation to achieve decarbonization is made easier. In an information processing method for an information processing apparatus including a controller, the controller is configured to acquire a measurement value of a physical quantity on greenhouse gas emissions, the measurement value being measured by a sensor at a plant for process manufacturing, acquire, based on the acquired measurement value, the amount of the greenhouse gas emissions integrated in a predetermined period, predict a future trend in the amount of the greenhouse gas emissions, based on a past trend, and control a display to display an image representing the past trend and the predicted future trend in the amount of the greenhouse gas emissions.

*FIG. 1*

**Description**

CROSS-REFERENCE TO RELATED APPLICATION

This application claims priority to Japanese Patent Application No. 2023-137610, filed on August 25, 2023, the entire contents of which are incorporated herein by reference.

TECHNICAL FIELD

The present disclosure relates to an information processing method, an information processing apparatus, an information processing system, and a program.

BACKGROUND

Information management systems that collect and consolidate various information on the operating conditions and the like of apparatuses constituting plants and provide such information to users such as operators and engineers are known. For example, Patent Literature (PTL) 1 describes technology for managing the operation and maintenance of waste treatment plants.

CITATION LIST

Patent Literature

PTL 1: JP 2003-308118 A

SUMMARY

Conventional information management systems in plants have room for improvement in terms of facilitating plant operation to achieve decarbonization.

It would be helpful to make plant operation to achieve decarbonization easier.

According to the present disclosure, an information processing method is:

(1) an information processing method for an information processing apparatus including a controller, wherein the controller is configured to:

acquire a measurement value of a physical quantity on greenhouse gas emissions, the measurement value being measured by a sensor at a plant for process manufacturing;
acquire, based on the acquired measurement value, an amount of the greenhouse gas emissions integrated in a predetermined period;
predict a future trend in the amount of the greenhouse gas emissions, based on a past trend; and
control a display to display an image representing the past trend and the predicted future trend in the amount of the greenhouse gas emissions.

Thus, in the information processing method, the image representing not only the past trend but also the predicted future trend in the amount of greenhouse gas emissions is displayed. Thus, a user (e.g., an on-site operator, system engineer, or the like) can know in advance when the amount of greenhouse gas emissions is likely to exceed a reference value, and can take necessary measures in advance in daily work. For example, the user can reduce waste due to excessive operation, leakage, and loss in equipment, deal with performance degradation due to deterioration of the equipment, and the like. Therefore, the information processing method can make plant operation to achieve decarbonization easier, even when the user lacks experience.

(2) In the information processing method according to (1), the controller may be configured to:

calculate, based on the acquired measurement value, energy consumption at the plant, for each production lot, production line, or device in the plant; and
acquire, based on the calculated energy consumption, the amount of the greenhouse gas emissions integrated in the predetermined period.

Thus, in the information processing method, the energy consumption at the plant is calculated for each production lot, production line, or device in the plant, and the amount of greenhouse gas emissions is acquired based on the energy consumption. Therefore, the information processing method can calculate the amount of greenhouse gas emissions with high accuracy.

(3) In the information processing method according to (1) or (2), the controller may be configured to notify an alarm when the predicted future trend in the amount of the greenhouse gas emissions becomes greater than the reference value.

Thus, the user can know in advance that the future trend in the amount of greenhouse gas emissions is likely to become greater than the reference value, and can take necessary measures in advance.

(4) In the information processing method according to any one of (1) to (3), the controller may be configured to control the display to display an image in which the past trend and the predicted future trend in the amount of the greenhouse gas emissions are organized by each energy flow or scope category.

Thus, the user can view information on the greenhouse gas emissions that is organized in a desired format and can take necessary measures.

(5) In the information processing method according to any one of (1) to (4), the controller may be configured to:

estimate, using the measurement value of the physical quantity measured by the sensor, a measurement value of the physical quantity at a location at which the physical quantity has not been measured by the sensor; and
acquire the amount of the greenhouse gas emissions integrated in the predetermined period, based on the measurement value of the physical quantity measured by the sensor and the estimated measurement value of the physical quantity.

Thus, in the information processing method, the amount of greenhouse gas emissions is acquired by estimating the measurement value of the physical quantity at the location at which the physical quantity has not been measured by the sensor. Therefore, the information processing method can provide information on the amount of greenhouse gas emissions even when sensors are not exhaustively located or not all the sensors work properly.

(6) In the information processing method according to any one of (1) to (5), the controller may be configured to:

calculate, based on the acquired measurement value, a diagnostic KPI related to the greenhouse gas emissions at a device used in the plant; and
control the display to further display an image representing the calculated diagnostic KPI.

Thus, in the information processing method, the diagnostic KPI related to the greenhouse gas emissions is calculated and displayed. Therefore, the user can easily identify a cause of generation of greenhouse gases and take necessary measures.

(7) In the information processing method according to any one of (1) to (6), the controller may be configured to control the display to display the image representing the past trend and the predicted future trend in the amount of the greenhouse gas emissions, so as to be distinguishable between energy consumption and energy loss.

Thus, in the information processing method, the information on the amount of greenhouse gas emissions is displayed so that the energy consumption and the energy loss can be distinguished. Therefore, the user can easily choose necessary measures to achieve the decarbonization as appropriate.

According to the present disclosure, an information processing apparatus is:
(8) an information processing apparatus configured to be able to communicate with a client apparatus, the information processing apparatus including a controller configured to:

acquire a measurement value of a physical quantity on greenhouse gas emissions, the measurement value being measured by a sensor at a plant for process manufacturing;
acquire, based on the acquired measurement value, an amount of the greenhouse gas emissions integrated in a predetermined period;
predict a future trend in the amount of the greenhouse gas emissions, based on a past trend; and
transmit, by a communication interface to the client apparatus, an image representing the past trend and the predicted future trend in the amount of the greenhouse gas emissions.

Thus, the information processing apparatus has the image representing not only the past trend but also the predicted future trend in the amount of greenhouse gas emissions displayed. Thus, the user can know in advance when the amount of greenhouse gas emissions is likely to exceed the reference value, and can take necessary measures in advance. Therefore, the information processing apparatus can make the plant operation to achieve decarbonization easier.

According to the present disclosure, an information processing system is:

(9) an information processing system including:

a client apparatus; and
an information processing apparatus configured to be able to communicate with the client apparatus, wherein
the information processing apparatus including a controller configured to:

acquire a measurement value of a physical quantity on greenhouse gas emissions, the measurement value being measured by a sensor at a plant for process manufacturing;
acquire, based on the acquired measurement value, an amount of the greenhouse gas emissions integrated in a predetermined period;
predict a future trend in the amount of the greenhouse gas emissions, based on a past trend; and
transmit, by a communication interface to the client apparatus, an image representing the past trend and the predicted future trend in the amount of the greenhouse gas emissions.

Thus, the information processing system has the image representing not only the past trend but also the predicted future trend in the amount of greenhouse gas emissions displayed. Thus, the user can know in advance when the amount of greenhouse gas emissions is likely to exceed the reference value, and can take necessary measures in advance. Therefore, the information processing system can make the plant operation to achieve decarbonization easier.

According to the present disclosure, a program is:
(10) a program configured to control an information processing apparatus configured to be able to communicate with a client apparatus, the information processing apparatus including a controller, the program configured to cause the controller to execute operations, the operations including:

acquiring a measurement value of a physical quantity on greenhouse gas emissions, the measurement value being measured by a sensor at a plant for process manufacturing;
acquiring, based on the acquired measurement value, an amount of the greenhouse gas emissions integrated in a predetermined period;
predicting a future trend in the amount of the greenhouse gas emissions, based on a past trend; and
transmitting, by a communication interface to the client apparatus, an image representing the past trend and the predicted future trend in the amount of the greenhouse gas emissions.

Thus, the information processing apparatus that is executed based on the program has the image representing not only the past trend but also the predicted future trend in the amount of greenhouse gas emissions displayed. Thus, the user can know in advance when the amount of greenhouse gas emissions is likely to exceed the reference value, and can take necessary measures in advance. Therefore, the program can make the plant operation to achieve decarbonization easier.

According to an embodiment of the present disclosure, it is possible to make plant operation to achieve decarbonization easier.

BRIEF DESCRIPTION OF THE DRAWINGS

In the accompanying drawings:

FIG. 1 is a diagram illustrating an example configuration of an information processing system according to an embodiment;
FIG. 2 is a block diagram illustrating an example configuration of a first server apparatus in FIG. 1;
FIG. 3 is a block diagram illustrating an example configuration of a second server apparatus in FIG. 1;
FIG. 4 is a block diagram illustrating an example configuration of a client apparatus in FIG. 1;
FIG. 5 is a flowchart illustrating an example of operations of the second server apparatus;
FIG. 6 is a flowchart illustrating an example of operations of the first server apparatus;
FIG. 7 is a flowchart illustrating an example of the operations of the first server apparatus;
FIG. 8 is a diagram illustrating an example of a screen displaying the amount of carbon dioxide emissions at a plant;
FIG. 9A is a diagram illustrating an example of a trend graph in the amount of carbon dioxide emissions;
FIG. 9B is a diagram illustrating an example of the trend graph in the amount of carbon dioxide emissions;
FIG. 10A is a diagram illustrating an example of a graph on rise/drop detection;
FIG. 10B is a diagram illustrating an example of the graph on rise/drop detection;
FIG. 11 is a diagram illustrating an example of a difference alarm graph;

FIG. 12 is a diagram illustrating an example of a graph for region monitoring;

FIG. 13 is a diagram illustrating an example of a graph on a variable line function;

FIG. 14 is a diagram illustrating an example of an edit screen for graph display;

FIG. 15 is a diagram illustrating an example of the edit screen for graph display;

FIG. 16 is a diagram illustrating an example of a graph indicating the breakdown of steam loss at the plant;

FIG. 17 is a diagram illustrating an example of a screen displaying steam consumption and loss in relation to steam flow paths in the plant; and

FIG. 18 is a diagram illustrating an example of a screen displaying the steam consumption and loss in relation to the steam flow paths in the plant.

## DETAILED DESCRIPTION

### <Comparative Example>

As a configuration according to a comparative example, PTL 1 (claim 1) states:
"An operation management method for a plurality of plants networked by connecting the plurality of plants to a computer in a management center, the operation management method comprising:

a data collection step of collecting various operation data on the plants from a data storage device in each plant to the computer in the management center by a communication means;

a diagnosis step of diagnosing, using the collected operation data, a status of each part in the plants at the time of collecting the data;

a remaining life calculation step of calculating a remaining life of each part in the plants by comparing an obtained diagnosis result with a diagnosis result of another plant stored in the computer in the management center; and

an operation condition notification step of determining, from the obtained remaining life of each part, an increase or decrease in the various operation data required to extend the remaining life, and notifying a control mechanism of the plant of the increase or decrease."

In recent years, because of climate change due to global warming caused by increase in greenhouse gases such as carbon dioxide, the increasing risk of depletion of fossil fuel resources, and the like, decarbonization efforts are made important in plant operations and in a series of business activities using plants. For example, efficient use of fossil fuel resources, reduction in the amount of carbon dioxide emissions, use of renewable energy (e.g., solar power and wind power), use of emissions trading, and the like are examples of such decarbonization efforts.

Specifically, for example, in manufacturing plants, power plants, and other plants (including factories), manufacturers use fossil fuels, such as oil and coal, as heat sources by burning the fossil fuels. In the power plants, heavy oil is burned to generate electricity and steam, which is then used to rotate steam turbines to generate electricity. In addition, trucks, ships, and the like used to transport products manufactured by the manufacturers also use the fossil fuels such as oil. The fossil fuels are also used in transportation of employees on business trips. Thus, in plant operation, the fossil fuels are used not only in the consumption of the fossil fuels in the plants, but also in a series of activities related to the plant operation, such as transportation of raw materials and products, and movement of related personnel. Therefore, in addition to ensuring the safe and stable operation of the plants, plant managers are also required to operate the plants so that greenhouse gas emissions are reduced to achieve decarbonization.

However, it may not always be clear to on-site users, such as operators and engineers, what operations of each device in the plants are related to carbon dioxide emissions and fossil fuel consumption, and what plant operation policies contribute to decarbonization. Therefore, it may be difficult for an on-site user, with only information provided by an information management system according to the comparative example, to manage a plant so that each device operates appropriately to achieve decarbonization. Thus, the information management system in the plant according to the comparative example has room for improvement in terms of facilitating plant operation to achieve decarbonization.

It would be helpful to make plant operation to achieve decarbonization easier.

### <Embodiment>

An embodiment of the present disclosure will be described below with reference to the drawings. In the drawings, portions having the same configurations or functions are denoted by the same reference numerals. In the description of the present embodiment, duplicate descriptions of the same portions may be omitted or simplified as appropriate.

(Information Processing System)

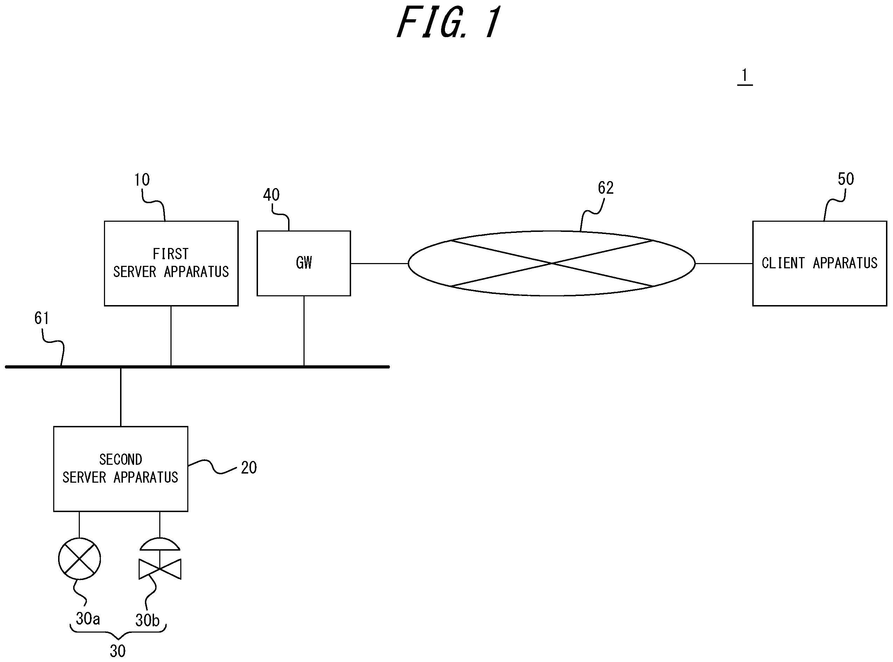

FIG. 1 is a diagram illustrating an example configuration of an information processing system 1 according to the embodiment. In the present embodiment, an example of a case in which the information processing system 1 is used in a plant for process manufacturing that uses fluids as raw materials will be described. Such a plant for the process manufacturing may include, for example, chemical plants, oil refineries, and the like. The information processing system 1 may also be used in plants other than those for such process manufacturing.

As illustrated in FIG. 1, the information processing system 1 includes a first server apparatus 10, a second server apparatus 20, sensors 30 (30a, 30b), a gateway 40, and a client apparatus 50. The sensors 30 (30a, 30b) are connected to the second server apparatus 20 communicably with each other. The first server apparatus 10, the second server apparatus 20, and the gateway 40 are communicably connected to each other via a network 61. The gateway 40 and the client apparatus 50 are communicably connected to each other via a network 62. The networks 61 and 62 may be, for example, an intranet, a dedicated network, the Internet, a mobile line, a combination thereof, or the like. The first server apparatus 10 and the client apparatus 50 may be able to communicate with each other via the same network (e.g., network 61).

The sensors 30 (30a, 30b) are devices that perform at least one of acquiring measurement values of physical quantities on greenhouse gas (e.g., carbon dioxide) emissions at the plant or operating the plant. The sensors 30 (30a, 30b) may include, for example, sensors 30a, such as temperature sensors, flow meters, or transmitters, and actuators 30b, such as valve devices, fans, or motors. Hereafter, the sensors 30a and 30b may be referred to collectively as "sensors 30". The sensors 30 transmit the measurement values to the second server apparatus 20, and control operations based on control signals received from the second server apparatus 20. In the example in FIG. 1, the information processing system 1 has two sensors 30a and 30b, but the number of sensors 30 is arbitrary.

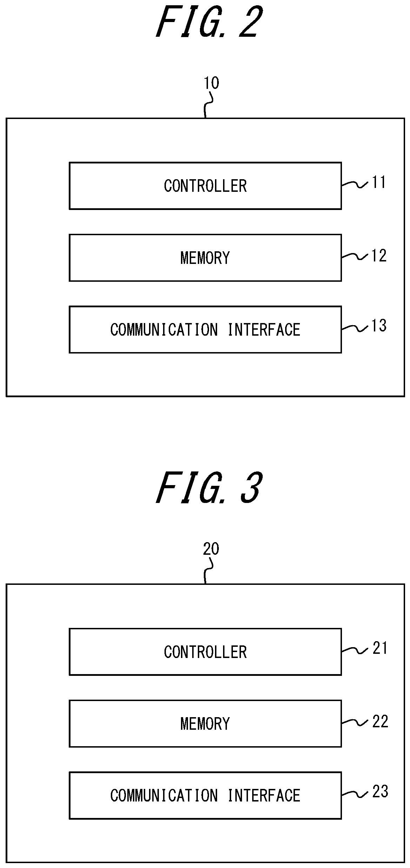

The second server apparatus 20, to which the sensors 30 are connected, functions as an interface between the sensors 30 and the first server apparatus 10. In other words, the second server apparatus 20 collects the measurement values of the physical quantities from the sensors 30 at regular intervals (e.g., every second). The second server apparatus 20 stores the collected measurement data in a memory 22 (see FIG. 3). The second server apparatus 20 transmits the measurement values of the sensors 30 to the first server apparatus 10 at regular intervals (e.g., every second). The second server apparatus 20 is connected to the network 61 and can communicate with the first server apparatus 10 via the network 61. In the example illustrated in FIG. 1, the information processing system 1 has one second server apparatus 20, but the number of second server apparatuses 20 is arbitrary. The number of sensors 30 connected to each second server apparatus 20 is arbitrary.

The first server apparatus 10, as an information processing apparatus according to the present embodiment, calculates, based on the measurement values of the physical quantities received from the second server apparatus 20, information on the amount of greenhouse gas emissions on plant operation, organizes the information into information that is easy for on-site users to understand, and provides the information to the client apparatus 50. The first server apparatus 10 is connected to the network 61 and can communicate with the second server apparatus 20 via the network 61. The first server apparatus 10 can communicate with the client apparatus 50 via the gateway 40 and the network 62. The information processing system 1 has one first server apparatus 10, but the number of first server apparatuses 10 is arbitrary.

The gateway 40 performs necessary protocol conversion, access control, and other processes to enable the apparatuses connected to the network 61 and the apparatuses connected to the network 62 to communicate with each other.

The client apparatus 50 is an apparatus operated by the on-site user such as an operator or engineer. The client apparatus 50 receives, from the first server apparatus 10, the information on the amount of greenhouse gas emissions on the plant operation, and displays the information.

In the above configuration, the first server apparatus 10 acquires the measurement values on the greenhouse gas emissions at the plant, which are measured by the sensors 30 in the plant. The first server apparatus 10 calculates, based on the acquired measurement values, energy consumption at the plant for each production lot, production line, or device in the plant. The first server apparatus 10 acquires, based on the calculated energy consumption, the amount of greenhouse gas emissions integrated in a predetermined period. The first server apparatus 10 predicts a future trend in the amount of greenhouse gas emissions, based on a past trend in the amount of greenhouse gas emissions. The first server apparatus 10 transmits, to the client apparatus 50, an image representing the energy consumption, the past trend in the amount of greenhouse gas emissions, and the predicted future trend in the amount of greenhouse gas emissions, and has the image displayed. The first server apparatus 10 notifies the client apparatus 50 of an alarm (warning) when the predicted future trend in the amount of greenhouse gas emissions exceeds a reference value.

Thus, the first the server apparatus 10 transmits, to the client apparatus 50, the image representing not only the energy consumption and the past trend in the amount of greenhouse gas emissions at the plant but also the predicted future trend. Therefore, the user can know in advance when the amount of greenhouse gas emissions may exceed the reference value and can take necessary measures.

The first server apparatus 10 displays the energy consumption at the plant so that the amount of energy consumed through use and the amount of energy consumed through loss (amount of energy discarded) can be distinguished. Thus, the user can easily and accurately grasp the status of energy use at the plant and can take necessary measures for more efficient energy use.

In addition to the information on the trends in the amount of greenhouse gas emissions and the like, the first server apparatus 10 also displays a diagnostic key performance indicator (KPI) related to the greenhouse gas emissions. The diagnostic KPI is information to facilitate identifying a cause of an equipment malfunction. The user can easily identify the cause of the equipment malfunction with reference to the diagnostic KPI, and take necessary measures such as replacement of parts and repairs.

The first server apparatus 10 also organizes the information on the amount of greenhouse gas emissions not only for each energy flow in the plant but also for each product supply chain, and provides the organized information to the client apparatus 50. At this time, the first server apparatus 10 also displays trends in the amount of carbon dioxide emissions in accordance with Scope 1-2-3 against emission limits, as well as monetary values according to carbon price and energy price, as appropriate. Thus, the user can grasp the status of the greenhouse gas emissions not only in the plant, but also in a series of activities related to the plant operation, such as transportation of raw materials and products and movement of related personnel, and can take necessary measures. To achieve such processing, the first server apparatus 10 may acquire not only measurement values measured at a particular plant, but also information on the amount of carbon dioxide emissions at each of preceding and subsequent processes of the plant. Specifically, the first server apparatus 10 may comprehensively calculate the amount of carbon dioxide emissions in real time, regardless of product, business type, region, and the like, from the accumulation of data on various business types, products, and supply chain networks collected from around the world. Furthermore, the first server apparatus 10 may visualize the amount of carbon dioxide emissions from various perspectives, such as per product or per factory, and may also predict and visualize the amount of carbon dioxide emissions in the future. This allows the user to consider and implement measures for decarbonization from various perspectives.

(First Server Apparatus)

FIG. 2 is a block diagram illustrating an example configuration of the first server apparatus 10 in FIG. 1. The first server apparatus 10 is one or more computer apparatuses that can communicate with each other. The first server apparatus 10 is, for example, any general purpose electronic device, such as a workstation (WS) or personal computer (PC), but may also be another dedicated electronic device. As illustrated in FIG. 2, the first server apparatus 10 includes a controller 11, a memory 12, and a communication interface 13.

The controller 11 includes one or more processors. In one embodiment, "processor" can be a general purpose processor, or a dedicated processor specialized for particular processing, but is not limited to this. The controller 11 is communicably connected to each component of the first server apparatus 10 and controls operations of the first server apparatus 10 as a whole.

The memory 12 includes, for example, any memory module such as a hard disk drive (HDD), solid state drive (SSD), read-only memory (ROM), and random access memory (RAM). The memory 12 may function as, for example, a main memory, an auxiliary memory, or a cache memory. The memory 12 stores any information used in the operations of the first server apparatus 10. For example, the memory 12 may store system programs, application programs, and various information received by the communication interface 13. The memory 12 is not limited to one built in the first server apparatus 10, but may be an external database or external memory module.

The communication interface 13 includes any communication module that can be connected to other devices, such as a scanner, by any communication technology. The communication interface 13 may further include a communication control module for controlling communication with the other devices and a memory module for storing communication data such as identification information required for communication with the other devices.

The functions of the first server apparatus 10 can be realized by executing a computer program (program) according to the present embodiment by a processor included in the controller 11. In other words, the functions of the first server apparatus 10 can be realized by software. The computer program causes a computer to perform processing of steps included in the operations of the first server apparatus 10, thereby causing the computer to realize the functions corresponding to the processing of each step. In other words, the computer program is a program to cause the computer to function as the first server apparatus 10 according to the present embodiment. The computer program may be recorded on a computer readable recording medium. A program includes information for processing by an electronic computer, and something equivalent to a program. For example, data that is not a direct command to a computer but has the nature of prescribing computer processing falls under the category of "something equivalent to a program".

Some or all of the functions of the first server apparatus 10 may be realized by a dedicated circuit included in the controller 11. In other words, some or all of the functions of the first server apparatus 10 may be realized by hardware. The first server apparatus 10 may be realized by a single computer or by the cooperation of multiple computers.

(Second Server Apparatus)

FIG. 3 is a block diagram illustrating an example configuration of the second server apparatus 20 in FIG. 1. The second server apparatus 20 is one or more computer apparatuses that can communicate with each other. The second server apparatus 20 is, for example, any general purpose electronic device, such as a WS or PC, but may also be another dedicated electronic device. As illustrated in FIG. 2, the second server apparatus 20 includes a controller 21, the memory 22, and a communication interface 23. The controller 21, memory 22, and communication interface 23 of the second server apparatus 20 are the same as the controller 11, memory 12, and communication interface 13 of the first server apparatus 10 described with reference to FIG. 2, so detailed descriptions are omitted. In the present embodiment, an example in which the first server apparatus 10 and the second server apparatus 20 are configured as separate apparatuses is described, but the first server apparatus 10 and the second server apparatus 20 may be configured as the same apparatus.

As with the first server apparatus 10, the functions of the second server apparatus 20 may be realized by software. Also, some or all of the functions of the second server apparatus 20 may be realized by a dedicated circuit included in the controller 21. In other words, some or all of the functions of the second server apparatus 20 may be realized by hardware. The second server apparatus 20 may be realized by a single computer or by the cooperation of multiple computers.

(Client Apparatus)

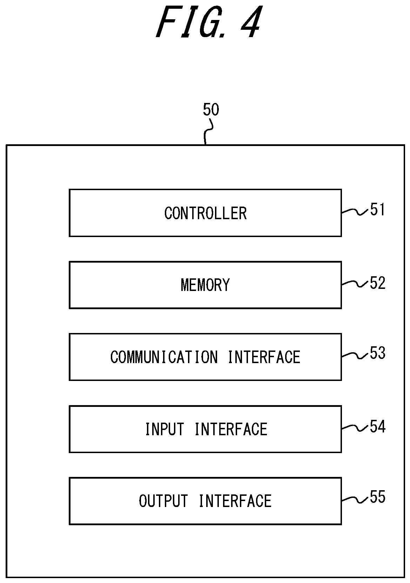

FIG. 4 is a block diagram illustrating an example configuration of the client apparatus 50 in FIG. 1. The client apparatus 50 is one or more computer apparatuses that can communicate with each other. The client apparatus 50 is any general purpose electronic device, such as a PC, tablet device, or smartphone, for example, but may also be another dedicated electronic device. As illustrated in FIG. 4, the client apparatus 50 includes a controller 51, a memory 52, a communication interface 53, an input interface 54, and an output interface 55. The controller 51, memory 52, and communication interface 53 of the client apparatus 50 are the same as the controller 11, memory 12, and communication interface 13 of the first server apparatus 10 described with reference to FIG. 2, so detailed descriptions are omitted.

The input interface 54 includes one or more input interfaces that accept user input operations and acquire input information based on the user operations. For example, the input interface 54 may be physical keys, capacitive keys, a pointing device, a touch screen integrated with a display of the output interface 55, a microphone that accepts voice input, or the like, but is not limited to these.

The output interface 55 includes one or more output interfaces that output information to the user and notify the user. For example, the output interface 55 can be a display that outputs information as an image, a speaker that outputs information as audio, or the like, but is not limited to these. Such a display may be, for example, a liquid crystal panel display or an organic electro luminescence (EL) display. At least one of the input interface 54 or the output interface 55 described above may be configured as an integral part of the client apparatus 50, or may be provided as a separate part.

In the present embodiment, an example in which the first server apparatus 10 and the client apparatus 50 are configured as separate apparatuses is described, but the first server apparatus 10 and the client apparatus 50 may be configured as the same apparatus.

As with the first server apparatus 10, the functions of the client apparatus 50 may be realized by software. Some or all of the functions of the client apparatus 50 may also be realized by a dedicated circuit included in the controller 51. In other words, some or all of the functions of the client apparatus 50 may be realized by hardware. The client apparatus 50 may be realized by a single computer or by the cooperation of multiple computers.

(Example of Operations)

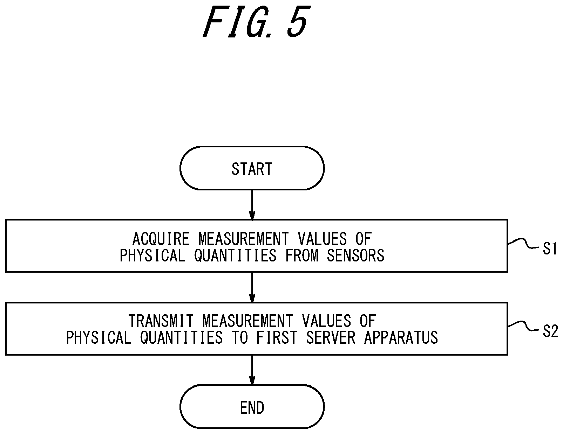

FIG. 5 is a flowchart illustrating an example of operations of the second server apparatus 20. The operations of the second server apparatus 20 described with reference to FIG. 5 may correspond to one of information processing methods of the second server apparatus 20. The operation of each step in FIG. 5 may be performed based on control by the controller 21 of the second server apparatus 20. The second server apparatus 20 may repeat a series of processes in FIG. 5 at any intervals (e.g., every second, every minute, or every hour). Since the second server apparatus 20 performs the series of processes at intervals that match the operating conditions of factory equipment, the user can easily notice excessive operation and waste such as leakage and loss. When a plurality of second server apparatuses 20 is provided in the information processing system 1, each of the second server apparatuses 20 may perform the processes of the flowchart in FIG. 5.

In step S1 of FIG. 5, the controller 21 acquires a measurement value of a physical quantity from each of the sensors 30 connected to the second server apparatus 20. Specifically, the controller 21 may receive and acquire the measurement value of the physical quantity from each of the sensors 30. The controller 21 may acquire the measurement values of the physical quantities from not only the sensors 30 directly connected to the second server apparatus 20, but also from the sensors 30 connected via the wired or wireless communication interface and any network.

In step S2, the controller 21 transmits the measurement values of the physical quantities received in step S1 to the first server apparatus 10. Then, the controller 21 ends the processes of the flowchart in FIG. 5.

Although FIG. 5 illustrates an example in which the timing of the second server apparatus 20 acquiring the measurement values of the physical quantities from the sensors 30 and the timing of the second server apparatus 20 transmitting the measurement values of the physical quantities to the first server apparatus 10 are synchronized, the timings may not be synchronized. For example, the second server apparatus 20 may transmit the measurement values of the physical quantities to the first server apparatus 10 in response to receiving a request from the first server apparatus 10.

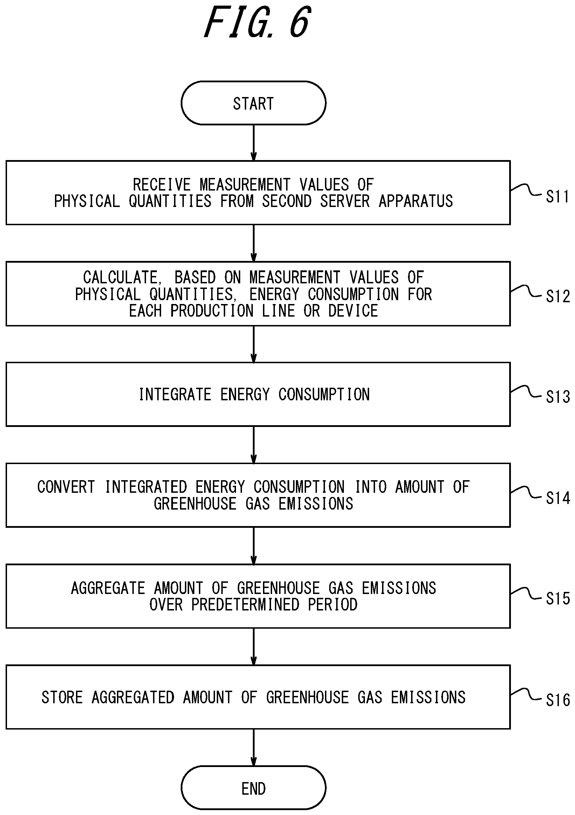

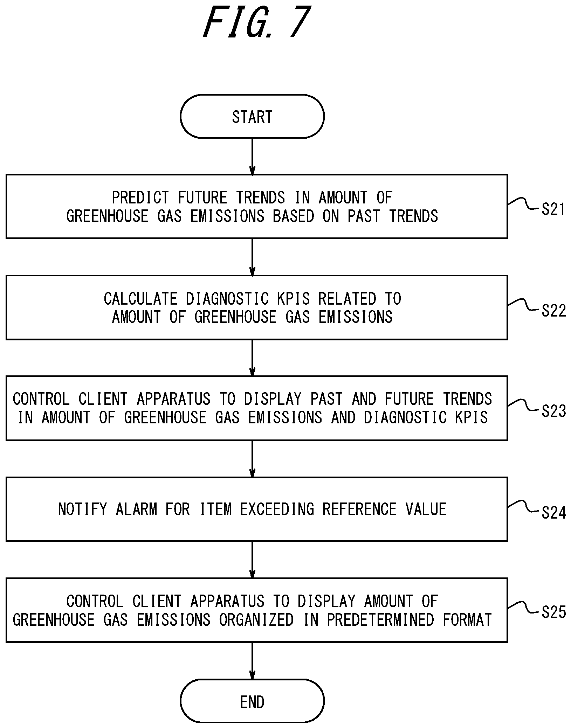

FIGS. 6 and 7 are flowcharts illustrating an example of operations of the first server apparatus 10. The operations of the first server apparatus 10 described with reference to FIGS. 6 and 7 may correspond to one of information processing methods of the first server apparatus 10. The operation of each step in FIGS. 6 and 7 may be performed based on control by the controller 11 of the first server apparatus 10.

FIG. 6 illustrates processes by which the first server apparatus 10 calculates, from the measurement values of the physical quantities, the energy consumption and the amount of greenhouse gas emissions. The first server apparatus 10 may repeat the series of processes in FIG. 6 at any intervals (e.g., every minute, every hour, everyday, every month, or every year).

In step S11 of FIG. 6, the controller 11 receives the measurement values of the physical quantities from the second server apparatus 20. The controller 11 thereby acquires the measurement value of each of the sensors 30.

In step S12, the controller 11 calculates, based on the measurement values of the physical quantities acquired in step S12, the energy consumption for each production lot, production line, or device. Specifically, the controller 11 converts each measurement value of the physical quantity into the energy consumption using a conversion expression predetermined according to the type of the physical quantity. The controller 11 may calculate the energy consumption so that the amount of energy actually consumed by operations of a device constituting the plant and the amount (loss) of energy lost due to exhaust or other reasons can be distinguished.

In step S13, the controller 11 integrates the energy consumption calculated in step S12. Specifically, the controller 11 calculates an integrated value of the energy consumption for each predetermined first period, such as minutes, hours, days, weeks, months, or years. As described above, the controller 11 repeats the process of each step in FIG. 6 at regular intervals. The controller 11 may update the integrated value by retaining the energy consumption and adding the energy consumption calculated in step S12 to the integrated value. When updating the integrated value results in the integrated value representing the integrated value of the energy consumption for the first period, the controller 11 may proceed to step S14, and then initialize the integrated value of the energy consumption to a value 0. This allows the controller 11 to calculate the integrated value of the energy consumption for each first period. The controller 11 may perform the processes after step S14 for each first period. Specifically, the controller 11 may proceed to step S14 only when the process of step S13 results in the integrated value representing the integrated value of the energy consumption for the first period, otherwise the processes of the flowchart in FIG. 6 for this cycle may be terminated.

In step S14, the controller 11 converts the integrated value of the energy consumption for each predetermined period calculated in step S13 into the amount of greenhouse gas emissions. Specifically, the controller 11 converts the integrated value of the energy consumption into the amount of greenhouse gas emissions using a predetermined conversion expression.

In step S15, the controller 11 aggregates the amount of greenhouse gas emissions over a predetermined period. Specifically, the controller 11 aggregates the amount of greenhouse gas emissions acquired in step S14 for each predetermined second period, such as days, weeks, months, or years. The second period is a longer period than or the same as the first period. Similar to the calculation of the integrated value of the energy consumption for each first period, the controller 11 may retain an integrated value of the amount of greenhouse gas emissions and add the amount of greenhouse gas emissions acquired in step S14 to the integrated value to update the integrated value. When updating the integrated value results in the integrated value representing the integrated value of the amount of greenhouse gas emissions for the second period, the controller 11 may proceed to step S16, and then initialize the integrated value of the amount of greenhouse gas emissions to a value 0. This allows the controller 11 to calculate the integrated value of the amount of greenhouse gas emissions for each second period. The controller 11 may perform the process of step S16 every second period. Specifically, the controller 11 may proceed to step S16 only when the process of step S15 results in the integrated value representing the integrated value of the amount of greenhouse gas emissions for the second period, otherwise the processes of the flowchart in FIG. 6 for this cycle may be terminated.

In step S16, the controller 11 stores, in the memory 12, the amount of greenhouse gas emissions aggregated in step S15. After completing the process in step S16, the controller 11 ends the processes of the flow chart in FIG. 6.

FIG. 7 illustrates processes by which the first server apparatus 10 transmits information on the amount of greenhouse gas emissions to the client apparatus 50, and has the information displayed. The first server apparatus 10 may perform the series of processes in FIG. 7 upon a request from the client apparatus 50. The first server apparatus 10 may also update the information at any time after displaying a screen on the client apparatus 50 by repeating the processes at arbitrary intervals (e.g., every minute, every hour, everyday, every month, or every year).

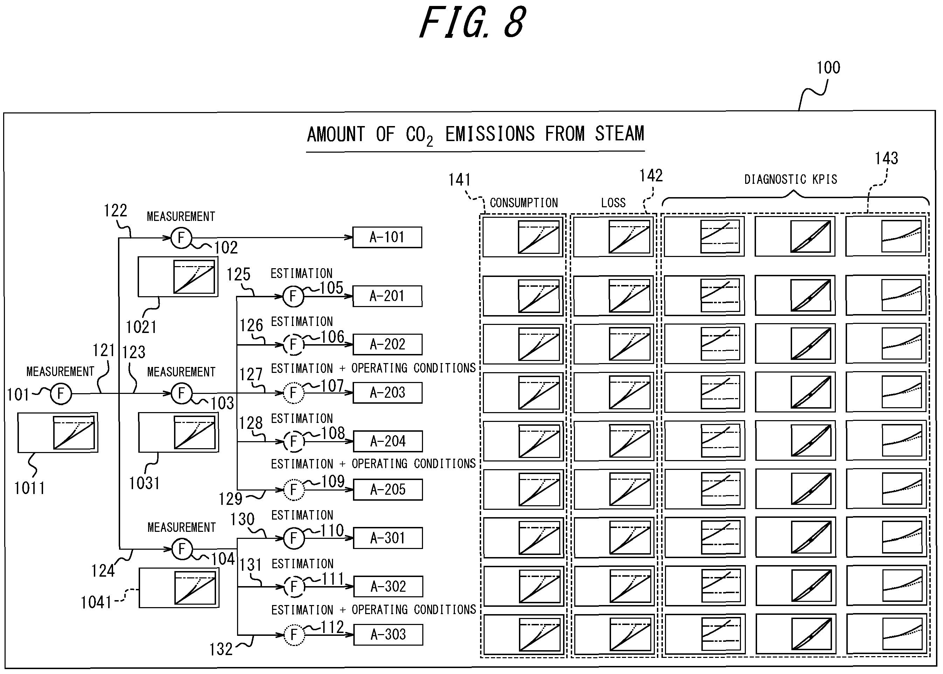

First, a brief overview of the screen to be displayed on the client apparatus 50 by the processes illustrated in FIG. 7 will be described. FIG. 8 is a diagram illustrating an example of a screen 100 illustrating the amount of carbon dioxide emissions at the plant. The screen 100 illustrates the amount of carbon dioxide ($CO_2$) emissions from steam at the plant that is a target of management by the information processing system 1.

In FIG. 8, figures "F" 101 to 112 indicate the locations of the sensors 30 (e.g., flow sensors, temperature sensors, pressure sensors, and the like) that measure a physical quantity related to steam, which can be converted into the amount of carbon dioxide emissions. Arrows 121 to 132 indicate flow paths of the steam. The following describes a case in which all of the sensors 30 indicated by the figures 101 to 112 measure flow rates of the steam. In the example in FIG. 8, the location of the sensor 30 of the figure 101 indicates the most upstream location of the flow paths of the steam. The arrow 121 of the flow path extending downstream from the location of the figure 101 branches into three flow paths indicated by the arrows 122 to 124. The flow path indicated by the arrow 123 branches into five flow paths indicated by the arrows 125 to 129. The flow path indicated by the arrow 124 branches into three flow paths indicated by the arrows 130 to 132. In FIG. 8, displays 1011 to 1041 illustrate energy consumption obtained by converting measurement values of physical quantity measured by the sensors 30 of the figures 101 to 104. The energy consumption is measured at multiple locations on an energy flow. The first server apparatus 10 may use data reconciliation operations to compensate for measurement errors of the sensors 30.

"A-101", "A-201", "A-202", ..., "A-303" indicate objects (equipment) to which the steam is supplied via the flow paths indicated by the arrows 121 to 132. A display 141 illustrates the energy consumption at each object. A display 142 illustrates the amount (loss) of energy discarded at each object. A display 143 illustrates diagnostic KPIs that indicate important information related to the energy consumption and loss at each object. The diagnostic KPIs are used to identify causes of the energy consumption and loss. Details of the information to be illustrated in the displays 141 to 143 are described below.

In the example in FIG. 8, "Measurement," "Estimation," and "Estimation + Operating Conditions" illustrated in the vicinity of the figures 101 to 112 indicate three states of the sensors 30. "Measurement" indicates that the physical quantity is actually measured by the sensor 30. "Estimation" indicates that the physical quantity is not actually measured by the sensor 30, but is estimated by dividing a measurement value of the sensor 30 in the vicinity thereof proportionally according to prorated values set in advance for each branch of the flow path. The prorated values may be indicated, for example, by a rate or percentage. "Estimation + Operating Conditions" indicates that the physical quantity is actually measured by the sensor 30 under certain conditions, and otherwise the physical quantity is estimated based on measurement values of the sensors 30 in the vicinity thereof.

For example, since the five flow paths indicated by the arrows 125 to 129 are branches of the flow path indicated by the arrow 123, the total value of flow rates of steam flowing through the five flow paths coincides with the measurement value of a flow rate by the sensor 30 of the figure 103. In other words, when flow rates at the sensors 30 of the figures 101, 102, ... are represented by $Q_{101}$, $Q_{102}$, ..., then $Q_{103} = Q_{105} + Q_{106} + Q_{107} + Q_{108} + Q_{109}$. Here, measurement values $I_{103}$ and $I_{105}$ of the flow rates $Q_{103}$ and $Q_{105}$ are measured by the sensors 30 indicated by the figures 103 and 105. Therefore, when the sensors 30 indicated by the figures 107 and 109 do not perform measurements, the controller 11 acquires estimated values of $Q_{106}$, $Q_{107}$, $Q_{108}$, and $Q_{109}$ by prorating ($I_{103}$ - $I_{105}$) by a predetermined ratio. Suppose that the sensor 30 indicated by the figure 107 performs a measurement and acquires a measurement value $I_{107}$, and the sensor 30 indicated by the figure 109 does not perform a measurement. In such a case, the controller 11 acquires estimated values of $Q_{106}$, $Q_{108}$, and $Q_{109}$ by prorating ($I_{103}$ - $I_{105}$ - $I_{107}$) by the predetermined ratio. Suppose that the sensor 30 indicated by the figure 109 performs a measurement and acquires a measurement value $I_{109}$, and the sensor 30 indicated by the figure 107 does not perform a measurement. In such a case, the controller 11 acquires estimated values of $Q_{106}$, $Q_{107}$, and $Q_{108}$ by prorating ($I_{103}$ - $I_{105}$ - $I_{109}$) by the predetermined ratio. When the sensors 30 indicated by the figures 107 and 109 perform measurements and acquire measurement values $I_{107}$ and $I_{109}$, the controller 11 acquires estimated values of $Q_{106}$ and $Q_{108}$ by prorating ($I_{103}$ - $I_{105}$ - $I_{107}$ - $I_{109}$) by the predetermined ratio.

Thus, the controller 11 estimates, using the measurement values of the sensors 30, the measurement values of the physical quantity at locations where no measurements have been performed by the sensors 30. The measurement value of the physical quantity in the flow path where no measurement has been performed may be estimated by a method other than that described above, depending on the type of physical quantity to be measured. For example, the controller 11 may estimate the measurement values of the physical quantity by correction by data reconciliation operations, material balance operations, or heat balance operations. The controller 11 may calculate the energy consumption by such estimation in step S12 of FIG. 6 for objects that have not been measured by the sensors 30.

Return to the explanation in FIG. 7. In step S21, the controller 11 predicts future trends in the amount of greenhouse gas emissions, based on past trends. The future trends in the amount of greenhouse gas emissions can be estimated based on any method. For example, the controller 11 may perform regression analysis on the past trends of the amount of greenhouse gas emissions and predict the future trends based on a regression curve.

In step S22, the controller 11 calculates the diagnostic KPIs related to the amount of greenhouse gas emissions. For example, pump performance, heat exchanger summary heat transfer coefficient, heating furnace load, distillation column return ratio, and reboiler load can be related to the amount of greenhouse gas emissions. Therefore, the sensors 30 may be installed to measure such physical quantities, and the controller 11 may calculate the diagnostic KPIs based on the measurement values. The user can use such diagnostic KPIs to identify a cause of excessive carbon dioxide emissions.

In step S23, the controller 11 controls the client apparatus 50 to display the past and future trends in the amount of greenhouse gas emissions acquired in step S21 and the diagnostic KPIs acquired in step S22. Specifically, the controller 11 generates images illustrating such information, transmits the images to the client apparatus 50, and controls a display, as the output interface 55 of the client apparatus 50, to display the information.

In step S24, the controller 11 notifies the user of an alarm for an item that exceeds a reference value. The controller 11 may notify the user of such an alarm by image highlighting or audio notification or the like.

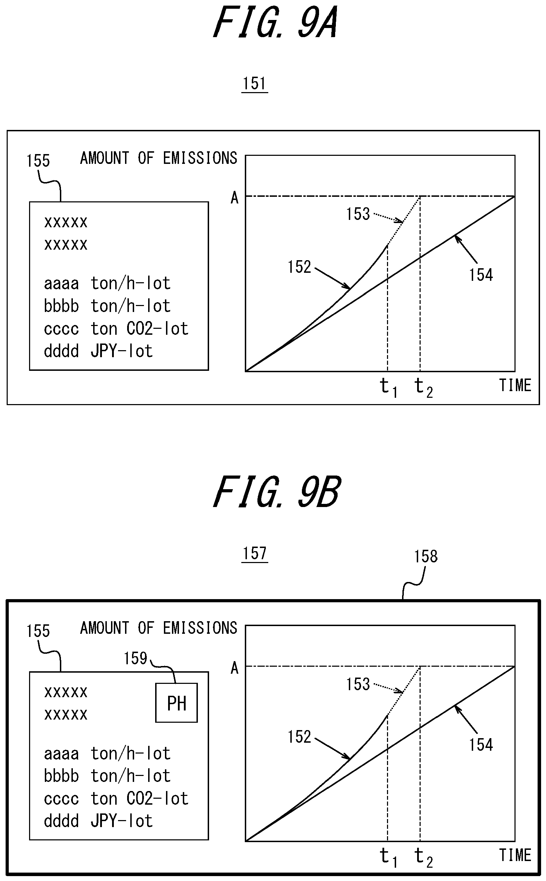

FIGS. 9A and 9B are diagrams illustrating an example of a trend graph in the amount of carbon dioxide emissions. FIGS. 9A and 9B illustrate an example of the past trend in the amount of greenhouse gas emissions and an estimated value of the future trend. FIG. 9B illustrates an example in which an image is highlighted when the greenhouse gas emissions are expected to exceed a reference value.

In an image 151 in FIG. 9A, the horizontal axis represents time, and the vertical axis represents the amount of carbon dioxide emissions. The amount of emissions on the vertical axis is expressed, for example, as an integrated value of carbon dioxide emissions, but may also be expressed as energy consumption or an amount corresponding to carbon price or energy price, depending on an user instruction or other factors. A graph 152 illustrates a past trend in the amount of carbon dioxide emissions from a start of measurement to a current time ti. A graph 153 illustrates a future trend in the amount of emissions estimated from the past trend in the amount of carbon dioxide emissions. A graph 154 illustrates a planned value (golden line) of the amount of carbon dioxide emissions, which indicates a trend as baseline carbon dioxide emissions. A display 155 illustrates information regarding various parameters in the graph 152.

In FIG. 9A, the graph 153 indicates that the amount of carbon dioxide emissions is expected to reach a reference value A at an earlier time $t_2$ than the graph 154 as a reference. Therefore, the controller 11 notifies the user of the alarm. FIG. 9B illustrates an image 157 to notify the user of the alarm by highlighting. Specifically, the image 157 notifies the user of the alarm by adding an outer frame 158 and a figure 159 to the image 151. A method of notifying the user of the alarm is not limited to that illustrated in FIG. 9B.

The controller 11 may display the trend graphs in the amount of carbon dioxide emissions as illustrated in FIGS. 9A and 9B for each sensor 30 (e.g., displays 1011 to 1041) or for each object (displays 141 and 142). In FIG. 8, "Consumption" (display 141) indicates the amount of carbon dioxide emissions corresponding to energy consumed. "Loss" (display 142) indicates the amount of carbon dioxide emissions corresponding to energy discarded at the plant. The "Loss" occurs, for example, when gas, steam, or the like is forcibly discharged for some reason from equipment, piping, or the like in the plant. These "Consumption," "Loss," and the like are calculated by converting the measurement value of the physical quantity measured by the sensor 30 into energy or carbon dioxide emissions using a pre-set conversion expression, conversion coefficient, and the like in processes in step S12 and later in FIG. 6.

In the example in FIG. 8, the controller 11 displays the trend graphs in the amount of carbon dioxide emissions for each sensor 30 and for each object, but the controller 11 may display trend graphs that are aggregated by different criteria. For example, the controller 11 may display trend graphs for each type of energy, such as electricity, steam, fuel, or the like, by aggregating the amount of carbon dioxide emissions relative to a product lot emission upper limit. Alternatively, as described below, the controller 11 may display trends in the amount of carbon dioxide emissions in Scope 1-2-3 relative to emission upper limits for each product supply chain.

As illustrated in FIG. 8, in the present embodiment, the controller 11 displays "Consumption" and "Loss" separately. Thus, the user can choose appropriate measures according to the form of energy consumption at the plant. For example, when the ratio of "Loss" is large, the user can take measures to reduce "Loss" by, for example, installing a steam trap.

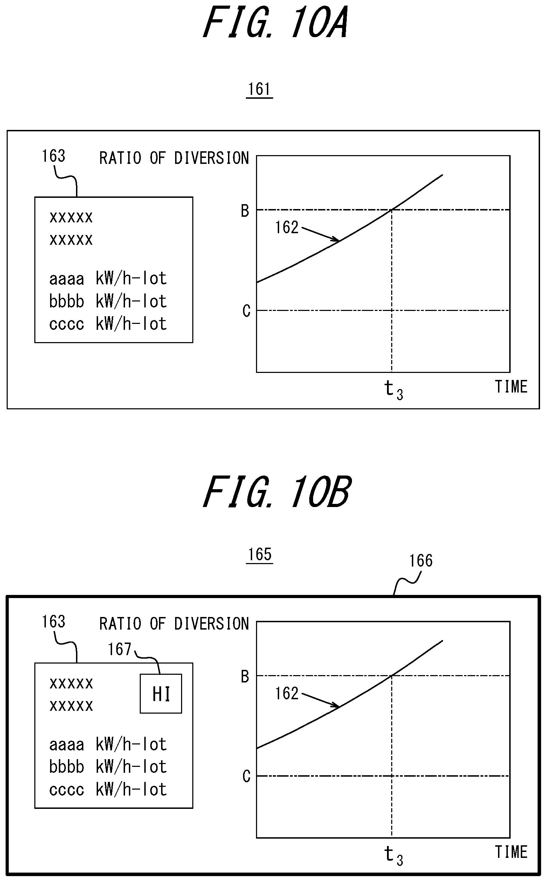

Next, examples of the diagnostic KPIs will be described with reference to FIGS. 10A to 13. FIGS. 10A and 10B are diagrams illustrating an example of a graph on rise/drop detection. FIGS. 10A and 10B illustrate an example of notifying the user of an alarm when a measurement value of an object to be monitored becomes smaller than a lower limit or larger than an upper limit. The graph on the rise/drop detection is suitable for detecting a phenomenon related to the degradation of performance (e.g., safety, availability, maintainability, and efficiency) associated with the deterioration of equipment when the equipment at the plant is used for long time.

For example, in a heat exchanger, tubes gradually become dirty as being used on a monthly basis. As the tubes become dirty, trends of energy consumption gradually worsen. Therefore, the sensors 30 for measuring steam temperature, raw material temperature, flow rate, and the like may be provided, and the controller 11 may calculate dirt of the tubes in the heat exchanger from measurement values of the sensors 30. By checking the calculated dirt, the user can ascertain that the dirt of the tubes is a cause of increase in the greenhouse gas emissions. The user can take measures such as cleaning or replacing parts when the dirt is significant.

Alternatively, the controller 11 may monitor the degradation of a distillation column. The distillation column is an apparatus that warms and distributes a raw material. For the distillation column, the controller 11 may acquire trends in a diversion ratio, i.e., a ratio of diversion of up and down flow rates by the sensors 30, and display the trends. By checking the calculated diversion ratio, the user can ascertain that a cause of increase in the greenhouse gas emissions is the deterioration of the distillation column. The on-site operator may apply heat, when the ratio is poor, with reference to the display of the ratio of diversion. The operator may also replace necessary parts, or the like when the ability of the distillation column, as expressed by the ratio of diversion, deteriorates significantly.

In an image 161 in FIG. 10A, the horizontal axis represents time, and the vertical axis represents the ratio of diversion of the distillation column. A graph 162 illustrates a time trend in the ratio of diversion. A display 163 illustrates information regarding various parameters of the graph 162. "B" indicates an upper limit of the ratio of diversion, and "C" indicates a lower limit of the ratio of diversion.

In FIG. 10A, the graph 162 illustrates that the ratio of diversion of the distillation column has reached the upper limit B at a time $t_3$. Therefore, the controller 11 notifies the user of an alarm. FIG. 10B illustrates an image 165 that notifies the user of the alarm by highlighting. Specifically, the image 165 notifies the user of the alarm by adding an outer frame 166 and a figure 167 to the image 161. A method of notifying the user of the alarm is not limited to that illustrated in FIG. 10B. By notifying the user of such an alarm, the user can know that the performance of the distillation column is deteriorating and can take necessary measures such as replacing parts or the like.

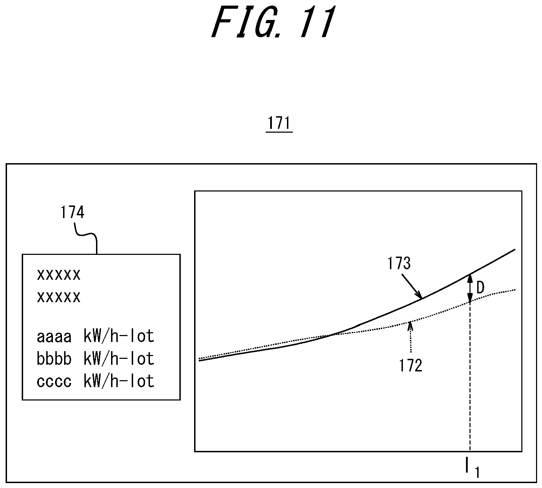

FIG. 11 is a diagram illustrating an example of a difference alarm graph. The differential alarm graph is used to compare two measurement values. For example, the difference alarm graph may be used to compare the design performance of equipment constituting the plant with a measurement value of the actual performance of the equipment in operation. Specifically, it is known that when a certain amount of voltage is applied to a pump, there is a certain relationship between a discharge pressure and a current value. Therefore, a current value applied to the pump and a discharge pressure are measured by the sensors 30, and it is found that the pump is degraded when the measured pressure is lower than a value corresponding to the current value.

In an image 171 of FIG. 11, the horizontal axis represents the current value, and the vertical axis represents the discharge pressure by the pump. A graph 172 illustrates the relationship between the current value and the measurement value of the actual discharge pressure by the pump to be monitored. A graph 173 illustrates the relationship between the current value and the design value of the discharge pressure by the pump to be monitored. A display 174 illustrates information regarding the various parameters in the graph 172. By reviewing the graphs 172 and 173, the user can ascertain that a cause of decrease in greenhouse gas utilization efficiency is due to decrease in the performance of the pump. The user can take measures such as cleaning or replacing parts when the performance of the pump is significantly degraded.

In the example of FIG. 11, there is a difference in pressure between the graphs 172 and 173 by a value D at a current value $I_1$. The controller 11 may notify the user of an alarm when such a difference becomes greater than a predetermined threshold value. By notifying the user of such an alarm, the user can know that the performance of the pump is deteriorating and can take necessary measures such as replacing parts. As a result, the user can take appropriate measures to reduce the greenhouse gas emissions.

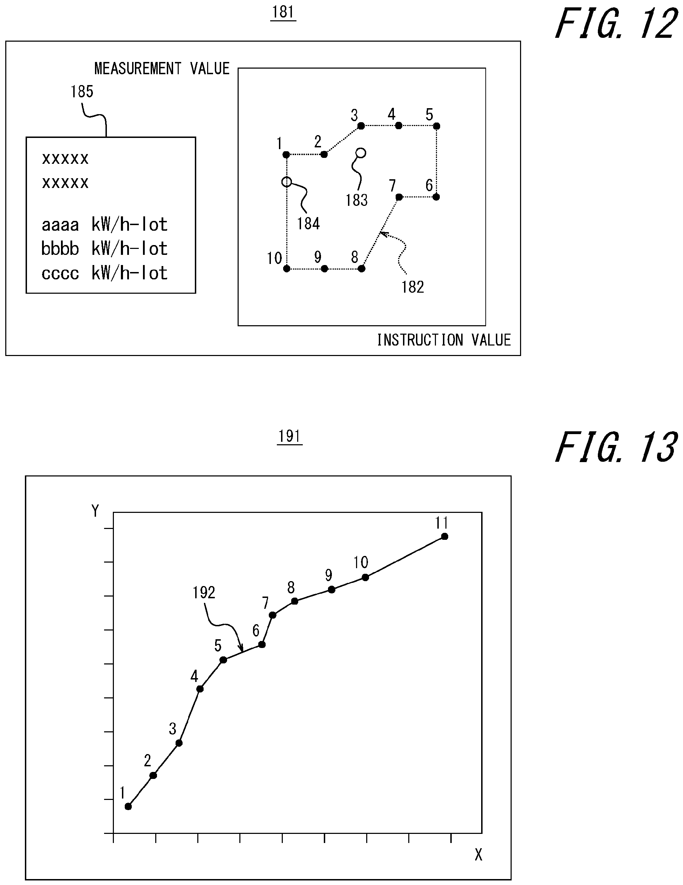

FIG. 12 illustrates an example of a graph for region monitoring. The graph for region monitoring is used to confirm that the relationship between two parameters related to an object exists within a certain region occupied in a two-dimensional plane. For example, in a normally operating control valve, it is known that an instruction signal (instruction value) X to the valve and an actual measurement value Y of movement of the valve in response to the signal take values within a certain region on the XY plane. However, as the valve deteriorates, the instruction signal X and the actual measurement value Y take values that deviate from the certain region. As a result, the valve for fuel flow may not open completely when the valve should open, or may not close completely when the valve should close. Therefore, the controller 11 can notify the user of an abnormality based on the deterioration of the valve in an easy-to-understand manner by display the deterioration of the valve as a graph of the region monitoring.

In an image 181 of FIG. 12, the horizontal axis represents the instruction value for the valve, and the vertical axis represents the actual measurement value of the actual movement of the valve. A graph 182 illustrates the certain region that defines the normal operation of the valve. Plots 183 and 184 each indicate an instruction value and an actual measurement value in the valve to be measured. The user can check whether the valve is operating normally by checking the plots 183 and 184 and the certain region indicated by the graph 182. When the plots 183 and 184 deviate from the certain region, the user can take necessary measures such as replacing the valve. The controller 11 may also notify the user of an alarm to prompt the user to replace the valve when the plots deviate from the certain region.

FIG. 13 is a diagram illustrating an example of a graph 192 on a variable line function. The variable line graph is used to compare two parameters. For example, the variable line graph may be used to compare the design performance X of equipment constituting the plant with a measurement value Y on the actual performance of the equipment in operation. The controller 11 may, for example, plot a measurement value of current applied to the pump as a value on the X axis and a measurement value of discharge pressure as a value on the Y axis, so that the user can see the relationship between the two in detail. As a result, the user can take appropriate measures to reduce the greenhouse gas emissions.

Although the examples of the diagnostic KPIs are described with reference to FIGS. 10A to 13, the controller 11 may provide information other than the information described above to the client apparatus 50 as a diagnostic KPI.

In step S25, the controller 11 controls the client apparatus 50 to display the amount of greenhouse gas emissions organized in a predetermined format. Specifically, the controller 11 may, for example, organize the amount of carbon dioxide emissions for each energy flow and display the trends. Alternatively, the controller 11 may, for example, organize the amount of carbon dioxide emissions for each factory energy type (e.g., electricity, steam, fuel, or the like), production lot, production process, factory equipment, or Scope 1-2-3 category (scope category), and display the trends. The controller 11 may organize the amount of greenhouse gas emissions in a format desired by the user according to the user's instruction. After completing the process in step S25, the controller 11 ends the processes of the flowchart in FIG. 7.

As described above, the first server apparatus 10 acquires the measurement values of the physical quantity on the greenhouse gas emissions measured by the sensor 30 at the plant for process manufacturing. The first server apparatus 10 acquires, based on the acquired measurement values, the amount of greenhouse gas emissions integrated in the predetermined period. The first server apparatus 10 predicts the future trend in the amount of greenhouse gas emissions, based on the past trend. The first server apparatus 10 transmits, to the client apparatus 50, the image illustrating the past trend and the predicted future trend in the amount of greenhouse gas emissions by the communication interface 13. Thus, the user can know in advance when the amount of greenhouse gas emissions may exceed the reference value, and can take necessary measures in advance. Thus, according to the first server apparatus 10, plant operation to achieve decarbonization can be made easier.

The first server apparatus 10 may also comprehensively calculate the amount of carbon dioxide emissions in real time, regardless of product, business type, region, and the like, from the accumulation of data on various business types, products, and supply chain networks collected from around the world. Furthermore, the first server apparatus 10 may visualize the amount of carbon dioxide emissions from various perspectives, such as per product or per factory, and may also predict and visualize the amount of carbon dioxide emissions in the future. This allows the user to consider and implement measures for decarbonization from various perspectives. The first server apparatus 10 may display, in response to the user choosing one of the graphs in the displays 141 to 143 in the screen 100 in FIG. 8 via the client apparatus 50, an enlarged view of the graph as illustrated in FIGS. 9A to 13.

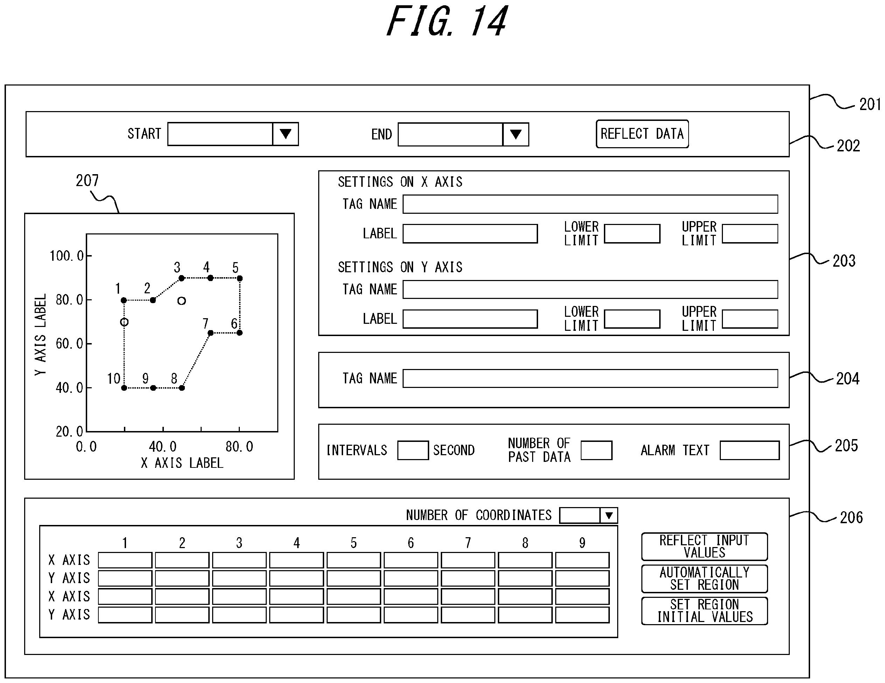

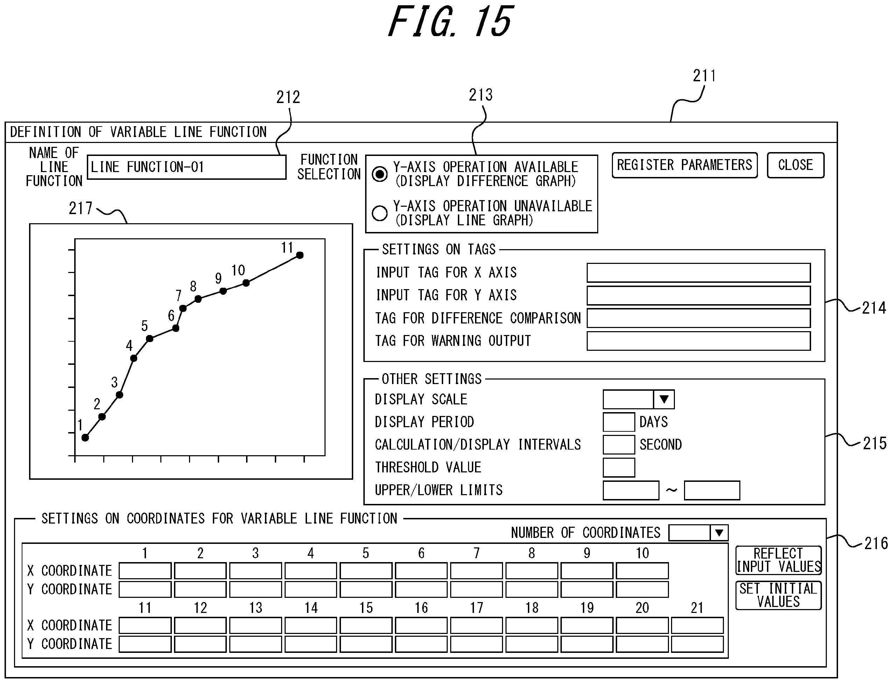

The first server apparatus 10 may provide the client apparatus 50 with an edit screen for information on the amount of carbon dioxide emissions, the diagnostic KPI, and the like calculated based on the measurement values of the sensor 30, and may accept edits from the user. FIGS. 14 and 15 are diagrams illustrating examples of the edit screen for graph display.

FIG. 14 illustrates an example of a screen 201 for setup of the graph for region monitoring. In the screen 201, an input area 202 is an area for setting a start period and an end period of data to be used for calculating the diagnostic KPI. An input area 203 is an area for setting the types, lower and upper limits, and the like of parameters to be set as the X and Y axes. An input area 204 is an area for setting a warning (alarm). An input area 205 is an area for setting data sampling intervals, the number of past data, and the like. An input area 206 is an area for setting a certain area in the two-dimensional plane. A display 207 is an area for displaying a preview screen of the graph for region monitoring.

FIG. 15 illustrates an example of a screen 211 for defining the variable line function. In the screen 211, an input area 212 is an area for setting a name to be applied to the line graph. An input area 213 is an area for selecting the function of the line graph. An input area 214 is an area for selecting tags to be set to the X-axis, Y-axis, and the like. An input area 215 is an area for setting a display scale, display period, calculation/display intervals, threshold value, and upper/lower limits. An area 216 is an area for setting coordinate values of the line function. A display 217 is an area for displaying a preview screen of the graph of the variable line function.

Thus, the first server apparatus 10 may provide the screens for editing the graph display, as illustrated in FIGS. 14 and 15. This allows the user to easily edit the screens for displaying the information on the amount of carbon dioxide emissions, the diagnostic KPI, and the like.

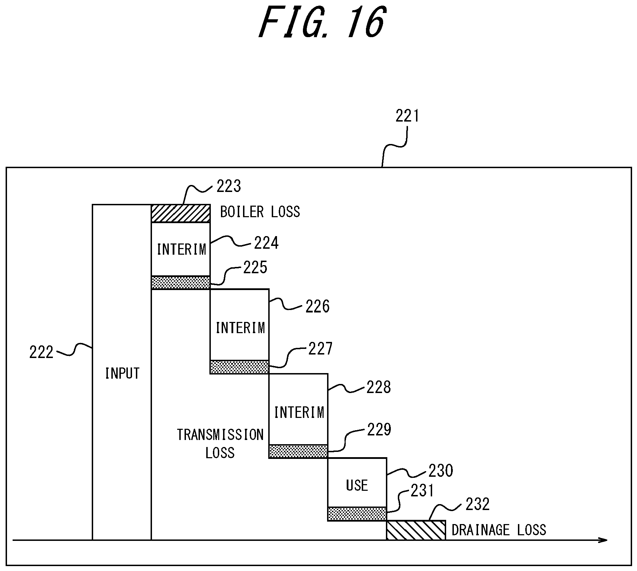

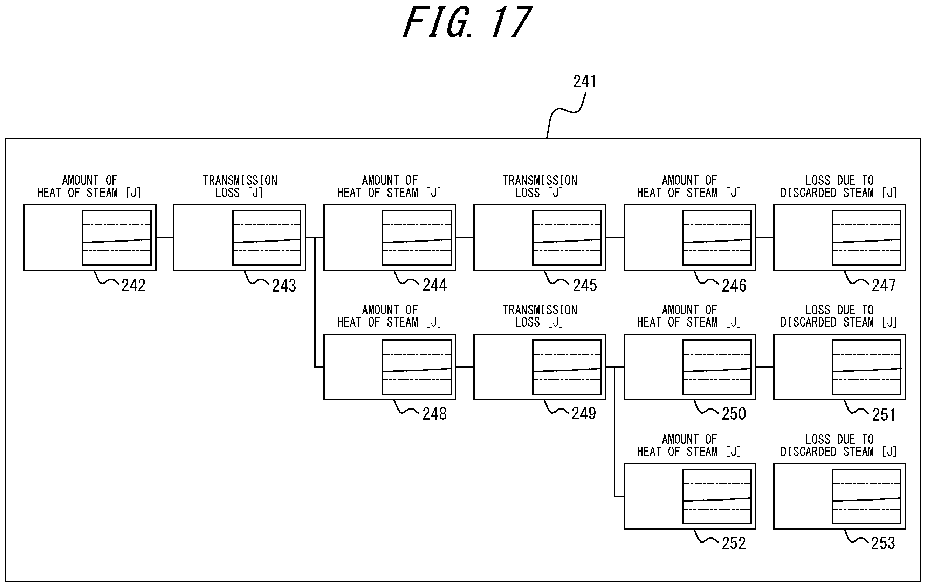

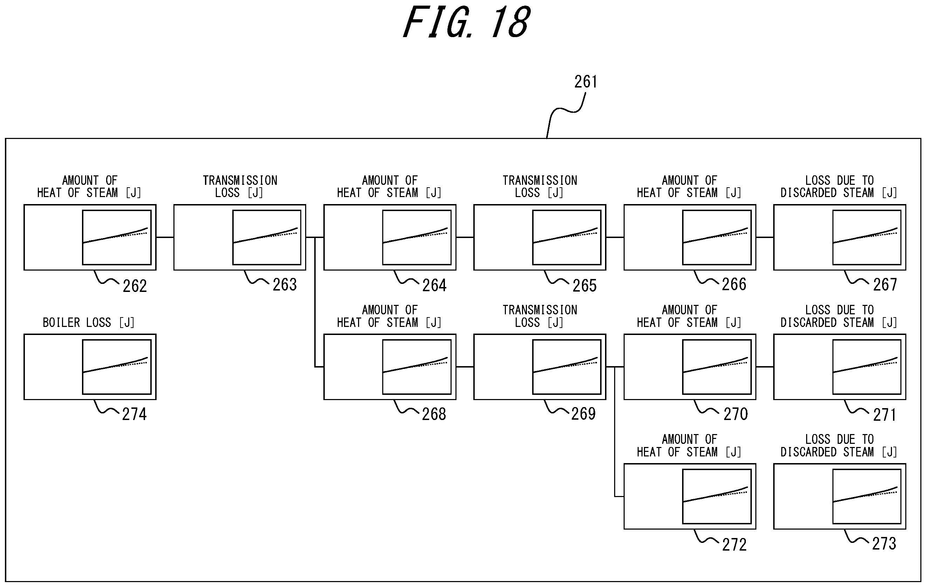

As described above, the first server apparatus 10 displays the energy consumption and loss in a distinguishable manner. The amount of energy loss can be calculated by multiple methods. The first server apparatus 10 may also display information regarding the amount of greenhouse gas emissions at the plant, including the energy loss, in various formats. As an example, a calculation method of the energy loss and the display of the information regarding the amount of greenhouse gas emissions at the plant, in which energy is transferred by steam heated by a boiler, will be further described. FIG. 16 is a diagram illustrating an example of a graph indicating a breakdown of steam loss at the plant. FIGS. 17 and 18 are diagrams illustrating an example of screens displaying steam consumption and loss in relation to steam flow paths in the plant.

When steam flowmeters are installed as the sensors 30 provided in such a plant, the information processing system 1 may calculate the consumption and loss of the steam using actual measurement values of the steam flowmeters. When no steam flowmeters are installed, the consumption and loss of the steam may be calculated based on measurement values of the other sensors 30. Methods of calculating the consumption and loss based on the measurement values of such other sensors 30 include, for example, a method of calculation based on a predetermined certain ratio, a method of calculation based on heat balance, and a method of calculation based on a relational expression between a cause of loss and the amount of loss. The energy consumption is measured at multiple locations in an energy flow. The first server apparatus 10 may use data reconciliation operations to correct for measurement errors in the sensors 30.

The method of calculation based on a predetermined certain ratio is, when a first flow path whose flow rate of steam has already been known is branched into multiple second flow paths as described above with reference to FIG. 8, a method of acquiring a flow rate of each of the second flow paths by prorating the flow rate of the first flow path by a predetermined ratio.

The method of calculation based on heat balance is a method of measuring the loss based on heat balance in a measurement target. In general, a heat $P_1$ of the measurement target is given as a value that is obtained by subtracting a heat loss $P_4$ from the sum of an externally given heat $P_2$ and a heat $P_3$ that the measurement target originally has. In other words, the following expression (1) is valid.

$$P_1 = P_2 + P_3 - P_4 \qquad (1)$$

The first server apparatus 10 may then calculate, using known values among $P_1$ to $P_4$, a remaining value in the expression (1).

The method of calculation based on a relational expression between a cause of loss and the amount of loss is a method in which the relationship between a measurement value of the sensor 30 related to the cause of loss and the amount of loss is measured in advance, and the amount of loss is measured using a relational expression representing the relationship. Such loss may include boiler loss, transmission loss, and drain loss, for example, but is not limited to these. The user may set up the edit screen for defining a variable line function, as described with reference to FIG. 15, and has the first server apparatus 10 acquire the variable line function representing the relationship between the cause of loss and the amount of loss. The first server apparatus 10 may calculate, using the variable line function, the amount of loss from the cause of loss. The following is a detailed description of examples of the method using such a relationship.

The boiler loss refers to the amount of heat lost in the boiler due to decrease in efficiency. When the recovery of latent heat of steam contained in exhaust gas is insufficient and the amount of latent heat is measured by the sensor 30, the first server apparatus 10 may calculate the boiler loss using an actual measurement value. When the amount of latent heat is not measured, the first server apparatus 10 may calculate the boiler loss by mathematization of the relationship between a damper opening degree and the amount of heat. To perform such a calculation, the user may set up the edit screen for defining a variable line function, as described with reference to FIG. 15, and have the first server apparatus 10 acquire the variable line function representing the relationship between the damper opening degree and the amount of heat. The first server apparatus 10 may calculate, using the variable line function, the boiler loss from the damper opening degree.

When the first server apparatus 10 does not measure the amount of latent heat, the first server apparatus 10 may calculate the loss in the boiler based on measurement values of other types of sensors 30. For example, when fuel is burned to generate steam in the boiler, it is known that there is a certain relationship between an air ratio, which is the ratio between fuel and air, and the amount of loss. Therefore, the first server apparatus 10 may mathematize in advance the relationship between the air ratio and the amount of loss, and calculate, using the mathematical expression, the amount of loss from the air ratio. It is also known that heat loss due to a high boiler water blow rate in the boiler has a certain relationship with boiler water. Therefore, the first server apparatus 10 may mathematize in advance the relationship between the boiler water and the amount of loss, and calculate, using the mathematical expression, the amount of loss from the boiler water. It is also known that loss based on insufficient air preheating has a certain relationship with preheating temperature. Therefore, the first server apparatus 10 may mathematize in advance the relationship between the preheating temperature and the amount of loss, and calculate, using the mathematical expression, the amount of loss from the preheating temperature. It is also known that loss caused by dirt of a burner installed in the boiler has a certain relationship with the degree of dirt. Therefore, the first server apparatus 10 may mathematize the relationship between the degree of dirt of the burner and the amount of loss with respect to a lapse of operation time, and calculate, using the mathematical expression, the amount of loss from the degree of dirt.

The transmission loss refers to the amount of heat lost in piping and valves. For example, for heat radiation loss from the piping, it is known that there is a certain relationship between the degree of peeling of a heat insulating material and the amount of loss with respect to a lapse of operation time. Therefore, the first server apparatus 10 may mathematize in advance the relationship between the degree of peeling of the heat insulating material in the piping and the amount of loss, and calculate, using the mathematical expression, the amount of loss in the piping from the degree of peeling. For heat radiation loss from the valves, it is also known that there is a certain relationship between the degree of peeling of a heat insulating material and the amount of loss with respect to a lapse of operation time. Therefore, the first server apparatus 10 may mathematize in advance the relationship between the degree of peeling of the heat insulating material in the valves and the amount of loss, and calculate, using the mathematical expression, the amount of loss in the valves from the degree of peeling.

The drain loss refers to loss from steam traps. The drain loss is caused by steam leakage or the like from valves, pipe connections, and the steam traps. It is known that loss due to the steam leakage from these parts become larger as operation time becomes longer. Therefore, the first server apparatus 10 may mathematize in advance the relationship between a lapse of operation time and the amount of loss for each of the valves, pipe connections, and steam traps, and calculate, using the mathematical expression, the amount of loss at the steam trap from the operation time.

Furthermore, the first server apparatus 10 may identify, as a KPI, a cause of increase or decrease in steam (or heat) consumption at each end unit (e.g., A-101, A-201, ... in FIG. 8). The first server apparatus 10 may mathematize the relationship between the KPI and the steam consumption in advance, and calculate, using the mathematical expression, the steam consumption at each end unit from the KPI. For example, when there is a certain relationship between the amount of increase and decrease in production of a product to be manufactured and increase and decrease in the amount of heat, the first server apparatus 10 may identify such a relationship in advance by a mathematical expression. The first server apparatus 10 may use such a mathematical expression to calculate increase or decrease in the amount of heat from the amount of increase or decrease in production of the product. Alternatively, the first server apparatus 10 may calculate, when the amount of heat of steam decreases, an impact on the amount of loss in the boiler using the aforementioned mathematical expression for the boiler loss. When there is a certain relationship between the amount of loss and measurement values related to disturbances such as outdoor temperature, indoor temperature, and tube dirt, the first server apparatus 10 may identify such a relationship in advance by a mathematical expression. The first server apparatus 10 may calculate, using such a mathematical expression, the impact on the amount of loss from the measurement values related to the disturbances. The first server apparatus 10 may also mathematize and identify the relationship between an empty operation state (e.g., switches of the units) and the amount of loss in advance. Such empty operation state includes, for example, supplying steam when it is not needed, such as when the line is stopped. The first server apparatus 10 can analyze, using such a mathematical expression, how the empty operation state affects the amount of loss.

The first server apparatus 10 may visually display the status of the energy consumption and loss at each area of a steam flow path, which are calculated as described above. FIG. 16 illustrates an example of a screen 221 that illustrates the status of the energy consumption and loss at each area of the flow path. The screen 221 illustrates the total amount of energy input to the plant and the amount of energy consumed or lost at the plant, in the form of a waterfall graph (waterfall chart). In FIG. 16, the height of an image 222 indicates the total amount of energy input to the plant.

In the example in FIG. 16, the screen 221 illustrates energy consumed and its breakdown at each of four areas in the steam flow path. In FIG. 16, images 223 to 225 indicate energy consumed at the first area of the flow path. The height of the image 223 indicates the amount of boiler loss. The height of the image 224 indicates the amount of energy consumed by a process in the first area. The height of the image 225 indicates the amount of transmission loss incurred in transmitting steam from the first area to the second area. Images 226 and 227 indicate energy consumed at the second area of the flow path. The height of the image 226 indicates the amount of energy consumed by a process in the second area. The height of the image 227 indicates the amount of transmission loss incurred in transmitting steam from the second area to the third area. Images 228 and 229 indicate energy consumed at the third area of the flow path. The height of the image 228 indicates the amount of energy consumed by a process in the third area. The height of the image 229 indicates the amount of transmission loss incurred in transmitting steam from the third area to the fourth area. Images 230 and 231 indicate energy consumed at the fourth area of the flow path. The height of the image 230 indicates the amount of energy consumed by a process in the fourth area. The height of the image 229 indicates the amount of transmission loss incurred when draining from the fourth area. An image 232 indicates the amount of drainage loss due to drainage. As illustrated in FIG. 16, the first server apparatus 10 may display the breakdown of the energy consumed at each area of the steam flow path. This allows the user to check the distribution of loss at each area and choose appropriate measures for decarbonization.

FIGS. 17 and 18 illustrate examples of screens displaying steam consumption and loss in relation to steam flow paths in the plant. In the plant for which FIGS. 17 and 18 illustrate the steam consumption and loss, the most upstream first flow path branches into a second flow path and a fourth flow path. The second flow path connects to a third flow path. The fourth flow path branches into a fifth flow path and a sixth flow path.

In FIG. 17, a screen 241 includes images 242 to 253. The image 242 illustrates the temporal transition of the amount of heat of steam consumed in the first flow path. The image 243 illustrates the temporal transition of the amount of transmission loss incurred when steam is transmitted from the first flow path to the second and fourth flow paths. The image 244 illustrates the temporal transition of the amount of heat of steam consumed in the second flow path. The image 245 illustrates the temporal transition of the amount of transmission loss incurred when steam is transmitted from the second flow path to the third flow path. The image 246 illustrates the temporal transition of the amount of heat of steam consumed in the third flow path. The image 247 illustrates the temporal transition of the amount of loss due to steam discarded in the third flow path. The image 248 illustrates the temporal transition of the amount of heat of steam consumed in the fourth flow path. The image 249 illustrates the temporal transition of the amount of transmission loss incurred when steam is transmitted from the fourth flow path to the fifth and sixth flow paths. The image 250 illustrates the temporal transition of the amount of heat of steam consumed in the fifth flow path. The image 251 illustrates the temporal transition of the amount of loss due to steam discarded in the fifth flow path. The image 252 illustrates the temporal transition of the amount of heat of steam consumed in the sixth flow path. The image 253 illustrates the temporal transition of the amount of loss due to steam discarded in the sixth flow path.

The images 242 to 253 each illustrate the relationship between lower and upper limits for a measurement value of the steam consumption or loss and the measurement value. As described with reference to FIG. 10B, the first server apparatus 10 may notify the user of an alarm when the measurement value of the steam consumption or loss becomes smaller than the lower limit or greater than the upper limit. Instead of displaying the measurement value of the steam consumption or loss so as to be comparable with both the upper and lower limits as illustrated in FIG. 17, the first server apparatus 10 may display the measurement value of the steam consumption or loss so as to be comparable with the upper limit as illustrated in FIGS. 9A and 9B. The first server apparatus 10 may display, in response to the user choosing one of the graphs in the images 242 to 253 in the screen 241 in FIG. 17 via the client apparatus 50, an enlarged view of the graph, as illustrated in FIGS. 9A to 13.

In FIG. 18, a screen 261 includes images 262 to 274. As described above, there are multiple methods for calculating the steam consumption and loss. The images 262 to 274 each display the steam consumption or loss measured by one of the multiple methods and the steam consumption or loss measured by another method. For example, the image 262 illustrates a time trend in the amount of heat of steam consumed in the first flow path. For example, the image 262 may display a measurement value of the amount of heat of steam consumed in the first flow path by a method of calculation based on heat balance and a measurement value by a method of calculation from a relational expression between a cause of loss and the amount of loss. The first server apparatus 10 may notify the user of an alarm when a difference between the values of the steam consumption or loss measured by the multiple methods becomes larger than a predetermined threshold value. This allows the user to recognize when an error between the methods for calculating the steam consumption and loss exceeds a certain value, verify a cause, and take appropriate measures.

In FIG. 18, the image 263 illustrates the temporal transition of the amount of transmission loss incurred when steam is transmitted from the first flow path to the second and fourth flow paths. The image 264 illustrates the temporal transition of the amount of heat of steam consumed in the second flow path. The image 265 illustrates the temporal transition of the amount of transmission loss incurred when steam is transmitted from the second flow path to the third flow path. The image 266 illustrates the temporal transition of the amount of heat of steam consumed in the third flow path. The image 267 illustrates the temporal transition of the amount of loss due to steam discarded in the third flow path. The image 268 illustrates the temporal transition of the amount of heat of steam consumed in the fourth flow path. The image 269 illustrates the temporal transition of the amount of transmission loss incurred when steam is transmitted from the fourth flow path to the fifth and sixth flow paths. The image 270 illustrates the temporal transition of the amount of heat of steam consumed in the fifth flow path. The image 271 illustrates the temporal transition of the amount of loss due to steam discarded in the fifth flow path. The image 272 illustrates the temporal transition of the amount of heat of steam consumed in the sixth flow path. The image 273 illustrates the temporal transition of the amount of loss due to steam discarded in the sixth flow path. The image 274 illustrates the temporal transition of the amount of loss of steam in the boiler.

Each of the images 263 to 274, as with the image 262, displays the steam consumption or loss measured by one of the multiple methods and the steam consumption or loss measured by another method. The first server apparatus 10 may notify the user of an alarm when, in any of the images 263 to 274, a difference between the values of the steam consumption or loss measured by the multiple methods becomes larger than a predetermined threshold value. The first server apparatus 10 may display, in response to the user choosing one of the graphs in the images 262 to 274 in the screen 261 in FIG. 18 via the client apparatus 50, an enlarged view of the graph, as illustrated in FIGS. 9A to 13.

As described above, the first server apparatus 10 displays the trend graphs illustrating the integrated value and the predicted value of the energy consumption. The trend graphs are updated at the regular intervals and display the integrated value in real time. Thus, the user can predict the amount of greenhouse gas emissions and take necessary measures. The first server apparatus 10 also displays the "planned value (golden line)" and "actual measurement value" of the amount of carbon dioxide emissions for each energy in the specified period. The first server apparatus 10 displays the line predicted from the actual measurement value and the planned upper limit, and displays an alarm when a predicted value exceeds the planned upper limit. Thus, the user can know in advance when the amount of greenhouse gas emissions is expected to exceed the planned upper limit, and can take necessary measures.

The first server apparatus 10 also displays the diagnostic KPIs that are useful in identifying a cause of energy consumption. The diagnostic KPIs may include, for example, the rise/drop detection, region monitoring, variable line function, and difference alarm graph. When any of these deviates from the reference value, the first server apparatus 10 notifies the user of an alarm, so that the user can accurately identify the cause related to the greenhouse gas emissions and take necessary measures.

The first server apparatus 10 also display a case of excessively using energy ("energy consumption") and a case of discarding energy ("energy loss") in a distinguishable manner. Thus, the user can distinguish between the consumption and lost, and take appropriate measures according to the respective causes.

As described above, according to the present embodiment, it is possible for the on-site operators and engineers in the manufacturing industry to achieve decarbonization easier in daily work, even when the on-site operators and

ЗЗ



**EP 4 513 396 A1**

engineers lack experience in decarbonization efforts.

The present disclosure is not limited to the embodiment described above. For example, a plurality of blocks illustrated in the block diagrams may be integrated, or one block may be divided. A plurality of steps illustrated in the flowcharts may be executed in parallel or in a different order, instead of being executed in chronological order according to the description, depending on the processing capability of the apparatus performing each step or as needed. Other variations may be possible to the extent of not departing from the intent of the present disclosure.

For example, the configuration and operations of the first server apparatus 10 may be distributed among multiple computers that can communicate with each other. For example, some or all of the components of the first server apparatus 10 may be provided in another device such as the second server apparatus 20.

## Claims

1. An information processing method for an information processing apparatus comprising a controller, wherein the controller is configured to:

   acquire a measurement value of a physical quantity on greenhouse gas emissions, the measurement value being measured by a sensor at a plant for process manufacturing;
   acquire, based on the acquired measurement value, an amount of the greenhouse gas emissions integrated in a predetermined period;
   predict a future trend in the amount of the greenhouse gas emissions, based on a past trend; and
   control a display to display an image representing the past trend and the predicted future trend in the amount of the greenhouse gas emissions.

2. The information processing method according to claim 1, wherein the controller is configured to:

   calculate, based on the acquired measurement value, energy consumption at the plant, for each production lot, production line, or device in the plant; and
   acquire, based on the calculated energy consumption, the amount of the greenhouse gas emissions integrated in the predetermined period.

3. The information processing method according to claim 1 or 2, wherein the controller is configured to notify an alarm when the predicted future trend in the amount of the greenhouse gas emissions becomes greater than a reference value.

4. The information processing method according to any one of claims 1 to 3, wherein the controller is configured to control the display to display an image in which the past trend and the predicted future trend in the amount of the greenhouse gas emissions are organized by each energy flow or scope category.

5. The information processing method according to any one of claims 1 to 4, wherein the controller is configured to:

   estimate, using the measurement value of the physical quantity measured by the sensor, a measurement value of the physical quantity at a location at which the physical quantity has not been measured by the sensor; and
   acquire the amount of the greenhouse gas emissions integrated in the predetermined period, based on the measurement value of the physical quantity measured by the sensor and the estimated measurement value of the physical quantity.

6. The information processing method according to any one of claims 1 to 5, wherein the controller is configured to:

   calculate, based on the acquired measurement value, a diagnostic KPI related to the greenhouse gas emissions at a device used in the plant; and
   control the display to further display an image representing the calculated diagnostic KPI.

7. The information processing method according to any one of claims 1 to 6, wherein the controller is configured to control the display to display the image representing the past trend and the predicted future trend in the amount of the greenhouse gas emissions, so as to be distinguishable between energy consumption and energy loss.

8. An information processing apparatus configured to be able to communicate with a client apparatus, the information

17

**EP 4 513 396 A1**

processing apparatus comprising a controller configured to:

acquire a measurement value of a physical quantity on greenhouse gas emissions, the measurement value being measured by a sensor at a plant for process manufacturing;
acquire, based on the acquired measurement value, an amount of the greenhouse gas emissions integrated in a predetermined period;
predict a future trend in the amount of the greenhouse gas emissions, based on a past trend; and
transmit, by a communication interface to the client apparatus, an image representing the past trend and the predicted future trend in the amount of the greenhouse gas emissions.

9. An information processing system comprising:

a client apparatus; and
an information processing apparatus configured to be able to communicate with the client apparatus, wherein
the information processing apparatus comprising a controller configured to:

acquire a measurement value of a physical quantity on greenhouse gas emissions, the measurement value being measured by a sensor at a plant for process manufacturing;
acquire, based on the acquired measurement value, an amount of the greenhouse gas emissions integrated in a predetermined period;
predict a future trend in the amount of the greenhouse gas emissions, based on a past trend; and
transmit, by a communication interface to the client apparatus, an image representing the past trend and the predicted future trend in the amount of the greenhouse gas emissions.

10. A program configured to control an information processing apparatus configured to be able to communicate with a client apparatus, the information processing apparatus comprising a controller, the program configured to cause the controller to execute operations, the operations comprising:

acquiring a measurement value of a physical quantity on greenhouse gas emissions, the measurement value being measured by a sensor at a plant for process manufacturing;
acquiring, based on the acquired measurement value, an amount of the greenhouse gas emissions integrated in a predetermined period;
predicting a future trend in the amount of the greenhouse gas emissions, based on a past trend; and
transmitting, by a communication interface to the client apparatus, an image representing the past trend and the predicted future trend in the amount of the greenhouse gas emissions.

18

# FIG. 1

EP 4 513 396 A1

# FIG. 2

10

| | |
|---|---|
| CONTROLLER | ∿ 11 |
| MEMORY | ∿ 12 |
| COMMUNICATION INTERFACE | ∿ 13 |

# FIG. 3

20

| | |
|---|---|
| CONTROLLER | ∿ 21 |
| MEMORY | ∿ 22 |
| COMMUNICATION INTERFACE | ∿ 23 |

# FIG. 4

50

| |
| --- |
| CONTROLLER ~51 |
| MEMORY ~52 |
| COMMUNICATION INTERFACE ~53 |
| INPUT INTERFACE ~54 |
| OUTPUT INTERFACE ~55 |

# FIG. 5

```
        ┌──────────────┐
        │    START     │
        └──────┬───────┘
               │
               ▼
┌──────────────────────────────────────────┐
│      ACQUIRE MEASUREMENT VALUES OF        │──── S1
│     PHYSICAL QUANTITIES FROM SENSORS      │
└──────────────────┬───────────────────────┘
                   │
                   ▼
┌──────────────────────────────────────────┐
│      TRANSMIT MEASUREMENT VALUES OF       │──── S2
│ PHYSICAL QUANTITIES TO FIRST SERVER APPARATUS │
└──────────────────┬───────────────────────┘
                   │
                   ▼
        ┌──────────────┐
        │     END      │
        └──────────────┘
```

# FIG. 6

START

RECEIVE MEASUREMENT VALUES OF
PHYSICAL QUANTITIES FROM SECOND SERVER APPARATUS — S11

CALCULATE, BASED ON MEASUREMENT VALUES OF
PHYSICAL QUANTITIES, ENERGY CONSUMPTION FOR
EACH PRODUCTION LINE OR DEVICE — S12

INTEGRATE ENERGY CONSUMPTION — S13

CONVERT INTEGRATED ENERGY CONSUMPTION INTO AMOUNT OF
GREENHOUSE GAS EMISSIONS — S14

AGGREGATE AMOUNT OF GREENHOUSE GAS EMISSIONS
OVER PREDETERMINED PERIOD — S15

STORE AGGREGATED AMOUNT OF GREENHOUSE GAS EMISSIONS — S16

END

# FIG. 7

```
                    ┌──────────────┐
                    │    START     │
                    └──────┬───────┘
                           │
                           ▼
   ┌─────────────────────────────────────────────────┐
   │        PREDICT FUTURE TRENDS IN AMOUNT OF        │   S21
   │  GREENHOUSE GAS EMISSIONS BASED ON PAST TRENDS   │
   └───────────────────────┬─────────────────────────┘
                           │
                           ▼
   ┌─────────────────────────────────────────────────┐
   │         CALCULATE DIAGNOSTIC KPIS RELATED TO     │   S22
   │         AMOUNT OF GREENHOUSE GAS EMISSIONS       │
   └───────────────────────┬─────────────────────────┘
                           │
                           ▼
   ┌─────────────────────────────────────────────────┐
   │ CONTROL CLIENT APPARATUS TO DISPLAY PAST AND     │   S23
   │ FUTURE TRENDS IN AMOUNT OF GREENHOUSE GAS        │
   │ EMISSIONS AND DIAGNOSTIC KPIS                    │
   └───────────────────────┬─────────────────────────┘
                           │
                           ▼
   ┌─────────────────────────────────────────────────┐
   │  NOTIFY ALARM FOR ITEM EXCEEDING REFERENCE VALUE │   S24
   └───────────────────────┬─────────────────────────┘
                           │
                           ▼
   ┌─────────────────────────────────────────────────┐
   │     CONTROL CLIENT APPARATUS TO DISPLAY AMOUNT   │   S25
   │ OF GREENHOUSE GAS EMISSIONS ORGANIZED IN         │
   │ PREDETERMINED FORMAT                             │
   └───────────────────────┬─────────────────────────┘
                           │
                           ▼
                    ┌──────────────┐
                    │     END      │
                    └──────────────┘
```

# FIG. 8

AMOUNT OF CO$_2$ EMISSIONS FROM STEAM

# FIG. 9A

<u>151</u>

155
AMOUNT OF EMISSIONS

xxxxx
xxxxx

aaaa ton/h-lot
bbbb ton/h-lot
cccc ton CO2-lot
dddd JPY-lot

A

153
152
154

t₁ t₂    TIME

# FIG. 9B

<u>157</u>    158

155
AMOUNT OF EMISSIONS
159

xxxxx    PH
xxxxx

aaaa ton/h-lot
bbbb ton/h-lot
cccc ton CO2-lot
dddd JPY-lot

A

153
152
154

t₁ t₂    TIME

# FIG. 10A

161

# FIG. 10B

165

166

# FIG. 11

171

FIG. 12

FIG. 13

# FIG. 14

# FIG. 15

**DEFINITION OF VARIABLE LINE FUNCTION** — 211, 212, 213

| | |
|---|---|
| NAME OF LINE FUNCTION | LINE FUNCTION-01 |

FUNCTION SELECTION
- ◉ Y-AXIS OPERATION AVAILABLE (DISPLAY DIFFERENCE GRAPH)
- ○ Y-AXIS OPERATION UNAVAILABLE (DISPLAY LINE GRAPH)

REGISTER PARAMETERS   CLOSE

217

**SETTINGS ON TAGS** — 214

| | |
|---|---|
| INPUT TAG FOR X AXIS | |
| INPUT TAG FOR Y AXIS | |
| TAG FOR DIFFERENCE COMPARISON | |
| TAG FOR WARNING OUTPUT | |

**OTHER SETTINGS** — 215

| | |
|---|---|
| DISPLAY SCALE | ▼ |
| DISPLAY PERIOD | DAYS |
| CALCULATION/DISPLAY INTERVALS | SECOND |
| THRESHOLD VALUE | |
| UPPER/LOWER LIMITS | ~ |

**SETTINGS ON COORDINATES FOR VARIABLE LINE FUNCTION** — 216

NUMBER OF COORDINATES ▼

| | 1 | 2 | 3 | 4 | 5 | 6 | 7 | 8 | 9 | 10 |
|---|---|---|---|---|---|---|---|---|---|---|
| X COORDINATE | | | | | | | | | | |
| Y COORDINATE | | | | | | | | | | |

| | 11 | 12 | 13 | 14 | 15 | 16 | 17 | 18 | 19 | 20 | 21 |
|---|---|---|---|---|---|---|---|---|---|---|---|
| X COORDINATE | | | | | | | | | | | |
| Y COORDINATE | | | | | | | | | | | |

REFLECT INPUT VALUES

SET INITIAL VALUES

EP 4 513 396 A1

# FIG. 16

# FIG. 17

EP 4 513 396 A1

# FIG. 18

EP 4 513 396 A1

Europäisches
Patentamt

European
Patent Office

Office européen
des brevets

# EUROPEAN SEARCH REPORT

**Application Number**

EP 24 19 4652

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/145629 A1 (BOTICH GEORGE [US] ET AL) 10 June 2010 (2010-06-10) * paragraph [0002] - paragraph [0136]; figures 1-17A * ----- | 1-10 | INV. G06Q10/04 G06Q10/06 G06Q50/04 G06Q50/06 |
| X | WO 2011/005863 A2 (SOUTH DAKOTA SCHOOL OF MINES AND TECHNOLOGY ET AL.) 13 January 2011 (2011-01-13) * paragraph [0143] - paragraph [0161] * ----- | 1-10 | |
| X | EP 2 244 216 A1 (ROCKWELL AUTOMATION TECH INC [US]) 27 October 2010 (2010-10-27) * paragraphs [0002] - [0007] - paragraph [0023]; figures 1-2 * ----- | 1-10 | |

**TECHNICAL FIELDS SEARCHED (IPC)**

G06Q

The present search report has been drawn up for all claims

| Place of search | Date of completion of the search | Examiner |
|---|---|---|
| Munich | 13 January 2025 | Pastore, Edoardo |

EPO FORM 1503 03.82 (P04C01)

## ANNEX TO THE EUROPEAN SEARCH REPORT
## ON EUROPEAN PATENT APPLICATION NO.

EP 24 19 4652

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2025

| Patent document cited in search report | | Publication date | Patent family member(s) | | Publication date |
|---|---|---|---|---|---|
| US 2010145629 | A1 | 10-06-2010 | BR | PI0912476 A2 | 24-09-2019 |
| | | | CA | 2724288 A1 | 19-11-2009 |
| | | | EP | 2283427 A1 | 16-02-2011 |
| | | | US | 2009281677 A1 | 12-11-2009 |
| | | | US | 2010145629 A1 | 10-06-2010 |
| | | | WO | 2009140314 A1 | 19-11-2009 |
| WO 2011005863 | A2 | 13-01-2011 | US | 2009287520 A1 | 19-11-2009 |
| | | | WO | 2011005863 A2 | 13-01-2011 |
| EP 2244216 | A1 | 27-10-2010 | CN | 101872190 A | 27-10-2010 |
| | | | EP | 2244216 A1 | 27-10-2010 |
| | | | US | 2010274602 A1 | 28-10-2010 |

EPO FORM P0459

For more details about this annex : see Official Journal of the European Patent Office, No. 12/82

**EP 4 513 396 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023137610 A

- JP 2003308118 A